# EUROPEAN PATENT SPECIFICATION

(11) **EP 3 188 650 B1**
(45) Date of publication and mention of the grant of the patent: **21.07.2021**
(21) Application number: 15837537.8
(22) Date of filing: 04.09.2015
(51) Int. Cl.: A61B 5/021, A61B 5/11, A61B 5/024, A61B 5/00

(54) **METHOD AND APPARATUS FOR THE CONTINOUS ESTIMATION OF HUMAN BLOOD PRESSURE USING VIDEO IMAGES**
VERFAHREN UND VORRICHTUNG ZUR KONTINUIERLICHEN SCHÄTZUNG DES MENSCHLICHEN BLUTDRUCKS ANHAND VON VIDEOBILDERN
PROCÉDÉ ET APPAREIL POUR L'ESTIMATION CONTINUE DE LA PRESSION SANGUINE HUMAINE AU MOYEN D'IMAGES VIDÉO

(30) Priority: 05.09.2014 US 201462046892 P
(43) Date of publication of application: 12.07.2017
(73) Proprietor: Lakeland Ventures Development, LLC, Grosse Pointe, Michigan 48230 (US)
(72) Inventor: WHITE, Craig William, Grosse Pointe, MI 48230 (US); AGUILAR-COUTINO, Artemio, Grosse Pointe, MI 48230 (US); VILLAREAL-GARZA, Procopio, Grosse Pointe, MI 48230 (US)
(74) Representative: Lloyd, Robin
(86) International application number: PCT/US2015/048491
(87) International publication number: WO 2016/037033

(56) References cited:
- US-A1- 2010 160 794
- US-A1- 2011 251 493
- US-A1- 2013 046 192
- US-A1- 2013 218 028
- US-A1- 2013 218 028
- US-A1- 2013 345 568
- US-A1- 2014 066 793
- US-A1- 2014 121 540
- US-A1- 2014 221 781
- BALAKRISHNAN GUHA ET AL: "Detecting Pulse from Head Motions in Video", IEEE COMPUTER SOCIETY CONFERENCE ON COMPUTER VISION AND PATTERN RECOGNITION. PROCEEDINGS, IEEE COMPUTER SOCIETY, US, 23 June 2013 (2013-06-23), pages 3430-3437, XP032492885, ISSN: 1063-6919, DOI: 10.1109/CVPR.2013.440 [retrieved on 2013-10-02]
- SHAN LI ET AL: "Video-based heart rate measurement using head motion tracking and ICA", 2013 6TH INTERNATIONAL CONGRESS ON IMAGE AND SIGNAL PROCESSING (CISP), IEEE, vol. 1, 16 December 2013 (2013-12-16), pages 160-164, XP032569259, DOI: 10.1109/CISP.2013.6743978 [retrieved on 2014-02-18]
- LI XIAOBAI ET AL: "Remote Heart Rate Measurement from Face Videos under Realistic Situations", 2014 IEEE CONFERENCE ON COMPUTER VISION AND PATTERN RECOGNITION, IEEE, 23 June 2014 (2014-06-23), pages 4264-4271, XP032649361, DOI: 10.1109/CVPR.2014.543 [retrieved on 2014-09-24]
- WIM VERKRUYSSE ET AL: "Remote plethysmographic imaging using ambient light", OPTICS EXPRESS, vol. 16, no. 26, 22 December 2008 (2008-12-22), page 21434, XP055193251, DOI: 10.1364/OE.16.021434
- WANG RUIPING ET AL: "Cuff-free blood pressure estimation using pulse transit time and heart rate", 2014 12TH INTERNATIONAL CONFERENCE ON SIGNAL PROCESSING (ICSP), IEEE, 19 October 2014 (2014-10-19), pages 115-118, XP032725440, ISSN: 2164-5221, DOI: 10.1109/ICOSP.2014.7014980 ISBN: 978-1-4799-2188-1 [retrieved on 2015-01-19]
- JOCHANAN E NASCHITZ ET AL: "Pulse Transit Time by R-Wave-Gated Infrared Photoplethysmography: Review of the Literature and Personal Experience", JOURNAL OF CLINICAL MONITORING AND COMPUTING, KLUWER ACADEMIC PUBLISHERS, DO, vol. 18, no. 5-6, 1 December 2004 (2004-12-01), pages 333-342, XP019250085, ISSN: 1573-2614
- None

## Description

### BACKGROUND

Through medical history, the use of the arterial blood pressure has been an important indicator of the state of the human health. Arterial blood pressure can also have other applications like the detection of the stress level on a subject or the indication that the subject is under the influence certain of substances.

Since the 18th century there have been instruments and methods to obtain a value that reflects the human artery blood pressure; however, many if not all of them rely on a direct contact with the subject under test. The invention described provides a way to use video image to estimate the blood pressure thus reducing or completely eliminating the need for human contact (non invasive). Since video images can be stored and transmitted, the estimation of the blood pressure can be performed locally, remotely and in real time or offline. US 2013/218028 A1 discloses a system and method for determining an arterial pulse transit time of a subject of interest in a remote sensing environment. BALAKRISHNAN GUHA ET AL: "Detecting Pulse from Head Motions in Video", IEEE COMPUTER SOCIETY CONFERENCE ON COMPUTER VISION AND PATTERN RECOGNITION, discloses extracting heart rate and beat lengths from videos by measuring head motion. US 2014/221781 A1 discloses a method and a device for monitoring at least one vehicle passenger in a vehicle involving capturing images of the vehicle passenger by an image capturing unit and analyzing the captured images using an image processing unit.

### SUMMARY OF THE INVENTION

The invention is defined by a method according to claim 1 and an apparatus according to claim 4.

### BRIEF DESCRIPTION OF THE DRAWINGS

**FIG. 1**. Describes in a block diagram the elements of an example embodiment to obtain the blood pressure estimation data.
**FIG. 2****.** Illustrates the steps involved in process that is performed by the Image Processing System in **FIG. 1****.**
**FIG. 3****.** Describes a more detailed flow diagram of the QRS pulse position estimation on the heart electrical signal cited in **FIG. 2****.**
**FIG. 4****.** Is a pictorial diagram that describes how the vertical movement signals are related to the human head.
**FIG. 5****.** Shows the continuation of the process that is described on **FIG. 2****.**
**FIG. 6****.** Is a flow diagram that describes the steps used in the preferred embodiment to obtain the image plethysmography in **FIG. 2****.**
**FIG. 7****.** Describes elements involved for the blood pressure estimation shown in **FIG. 2****.**
**FIG. 8****.** Shows with detail the blood pressure estimation model in **FIG. 7****.**

### DETAILED DESCRIPTION OF THE INVENTION

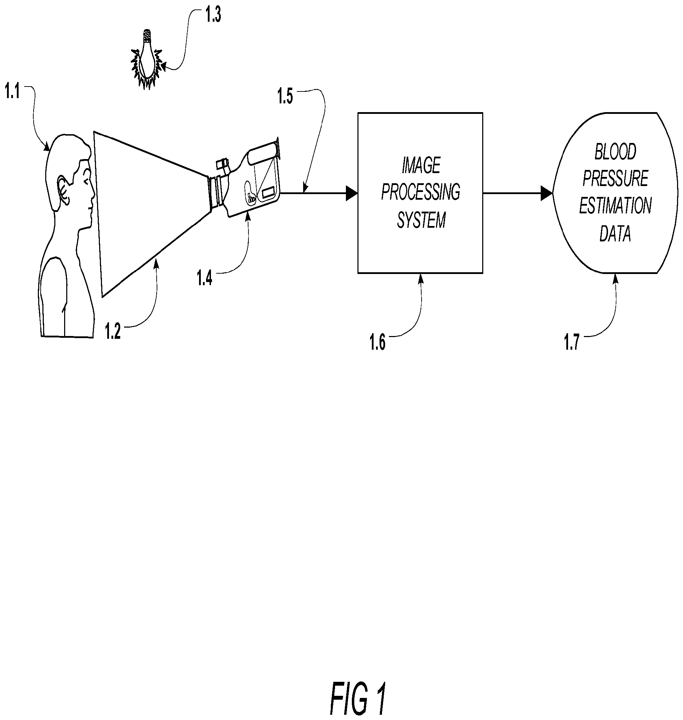

An example embodiment is shown in FIG 1. The later pictorial shows a human subject (1.1) properly illuminated by a stable light source (1.3) that can be natural ambient light from the sun or any type of artificial light that will provide the levels required for the video capture element (1.4). Light sources (1.3) with the enhancement of certain wavelengths can also be used if this benefits the image processing that will performed on (1.6).

The human subject (1.1) should be properly placed in front of the camera (1.4) so the field of view (1.2) includes the head of the subject (1.1) since for this example embodiment the head will contain the area of interest. Regarding the camera 1.4, a medium quality color "webcam" with a resolution of 1280 horizontal by 720 vertical pixels was used in one embodiment but lower quality images can also be employed. The frame rate chosen for the example embodiment was 30 frames per second using a non-compressed AVI format that is inputted using the camera interface (1.5) to the Image Processing System (1.6) using USB as the interface protocol. Using higher frame rates and resolution can improve the performance of the system.

Image Processing System (1.6) in this embodiment is implemented by a personal desktop computer. This system (1.6) can be implemented in any device (general purpose or embedded) that provides enough computational and processing power to perform the algorithms that comprise the process to estimate the blood pressure. Devices such as remote servers, smart phones, tablets or even implementations in hardware like FPGA's or ASICS are also acceptable ways for the implementation of the system (1.6). The system can also be entirely integrated in a single device so elements like the camera (1.4), camera interface (1.5) and the processing system (1.6) can be part of a "single" apparatus to the eyes of the user.

Regarding the video coupling (1.5) the video can be captured on another location different where the image processing system (1.6) resides so the video can be recorded and transmitted using conventional communication channels and protocols like the internet. This can be done using live streaming or previously recorded video files so is possible that the image processing system (1.6) operates on a real time basis or using a batch style processing, and the processing can be done for a single or a plurality of video sources (1.4).

At the output of the Image Processing System (1.6) there will be the data (1.7) that estimates the blood pressure of the subject (1.1) analyzed by (1.6). This data can be presented to a user via a screen display or can be any type of data storage or transmission element not necessarily used for human visualization. (1.7) can be used as the only value of interest or can be communicated to another system for further processing, transmission storage or visualization.

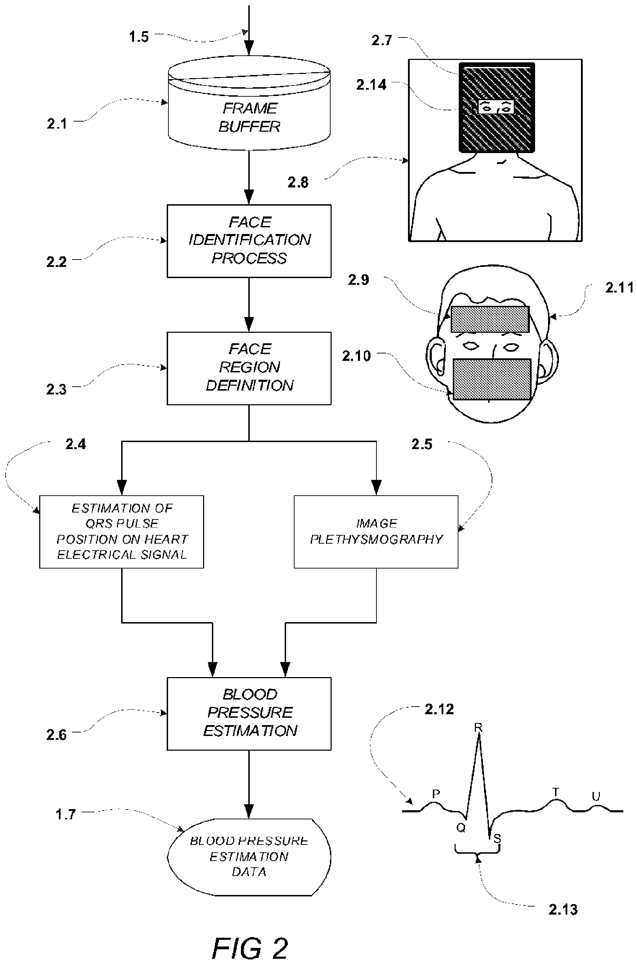

The video provided by interface (1.5) is stored on what is named a frame buffer (2.1) shown on FIG. 2. This frame buffer (2.1) as the name implies, stores an amount of video frames (2.8) so the algorithms that will be later executed have enough data for the operation. In this embodiment the value of 10 seconds of storage (∼300 frames) was used but this value can be changed depending on the type of subject (1.1) or the logical implementation of the algorithms and it can be optimized since it has a dependence on the way the architecture, code and language are used for the implementation. In an example embodiment, we used a "batch" style implementation that analyses the buffer and outputs the blood pressure data (1.7) but a real time approach can be used so the frame buffer (2.1) size can vary responsive to the analysis approach.

From each frame (2.8), the face identification process (2.2) implements an algorithm that eliminates the rest of the information from each frame (2.8) leaving only information related to the head (2.7) and also eliminates the area corresponding the eyes (2.14). Since the further processing will not use the removed information, the amount of data can be optimized and reduce the processing burden of the next stages of the processes. There are many public domain algorithms available and known to perform the face identification process. (2.2).

Once the image of the head (2.11) from the subject (1.1) is isolated from the rest of the frame (2.8); the first frame is used to define the regions on the head (2.11) that will be further isolated from the image. The algorithm selects two areas; below the eyes (2.10) and above the eyes (2.9). These areas are of particular interest since they have less border variation. Regarding the zone (2.10) further sections of the face can be eliminated like the nose and lips. At the end, what is desired are regions of the face that have homogenous pattern and that adequately reflect the light (1.3) so the signals that will result contain an acceptably small amount of noise.

In this example embodiment, there is no tracking algorithm used for the head (2.11). The latter implies that the method (2.3) to obtain the regions (2.9) and (2.10) requires that the subject (1.1) remains acceptably still and inside the field of view (1.2) of the camera (1.4) during the duration of the video capture. Those knowledgeable on the image processing discipline will appreciate that an object tracking algorithm can be implemented eliminating the stillness requirement of the subject (1.1).

The zones of interest (2.9) and (2.10) will be used as the input of two image processing algorithms. One is what we have called the Image Plethysmograpy (2.5) where the image data is processed to obtain a signal that represents the blood flow on the skin of the subject given the change of its volume as it will be further explained. The other block numbered (2.4) will estimate the location of the QRS pulse position on the heart electrical signal (2.12).

The human heart generates an electrical signal 2.12 that stimulates the movement of the heart muscles. The signal has a well known shape described on 2.12 and there is a peak located on what is called the QRS region (2.13). This peak is related to the moment of the maximum blood flow out of the heart and its position in time is required by the Blood Pressure Estimation process (2.6) to generate the resulting value of the analysis (1.7).

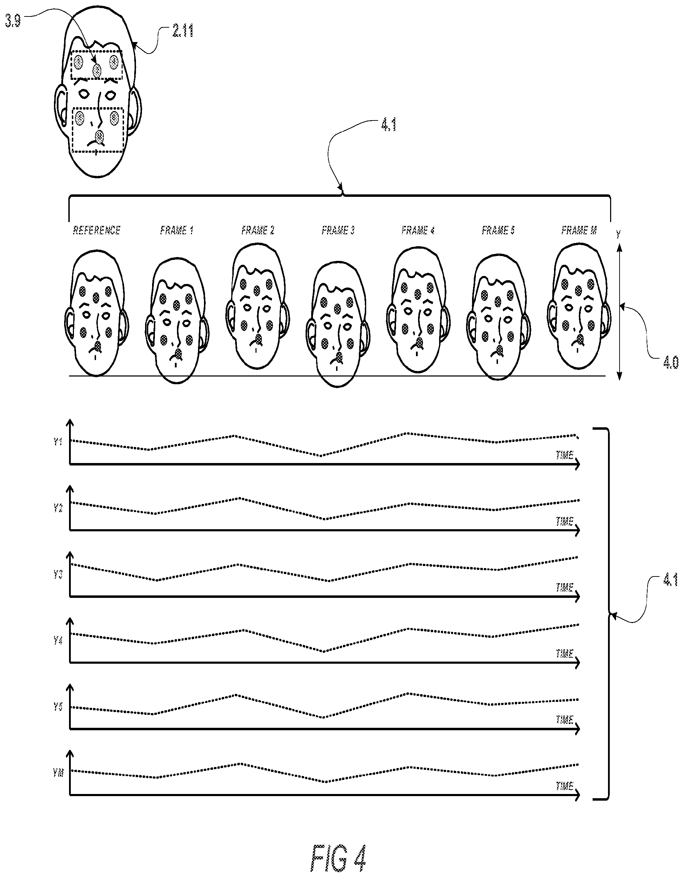

It is also known that when the heart pumps blood to the arteries, on the average human, the volume of blood pumped to the head is 25% of the total. This high percentage of blood containing oxygen is mainly directed to the brain via the carotid arteries. Since the blood flow is directed in the vertical axis of the head on a standing subject (1.1) and the volume of the blood is relatively high with respect to the size of the head (2.11) versus the rest of the body of the subject (1.1); the head (2.11) will move mainly vertically at the same rate the heart pumps the blood trough the arteries. It is obvious that this movement is imperceptible on the majority of subjects (1.1), but there are in fact pathological cases where the head movement on the subject is highly noticeable when they suffer from a disease called "aortic vascular insufficiency". In this embodiment, the process for the estimation of the position of the heart electrical signal (2.4) will be derived from this imperceptible vertical movement (4.0) on FIG 4.

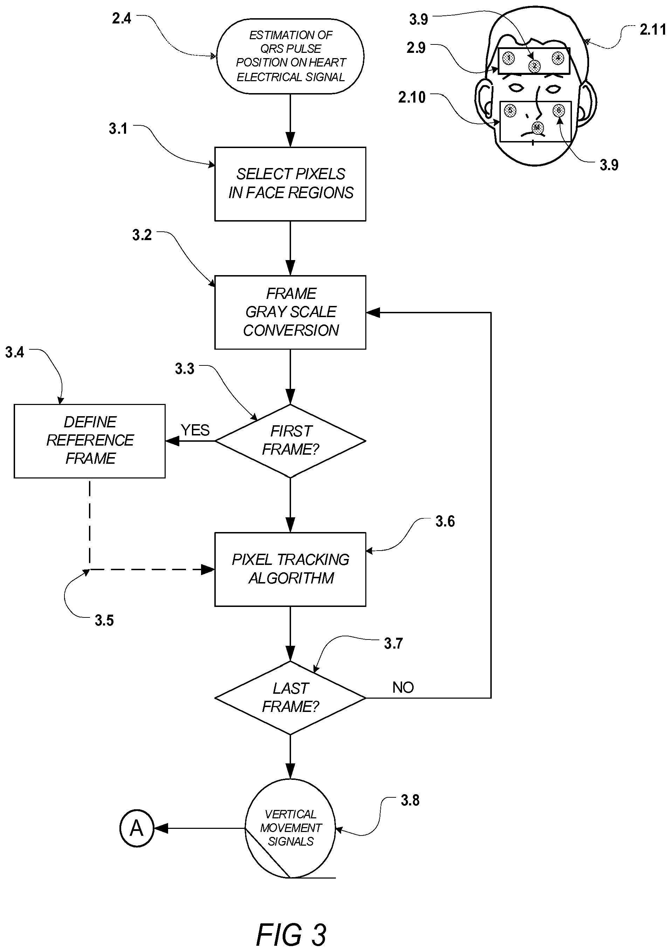

The process (2.4) used for estimation of the QRS pulse position on the heart electrical signal is described using a flow diagram on FIG. 3. The first step to this process (3.1) is to select a certain amount of pixels (3.9) on the regions (2.9) and (2.10) that were previously defined in (2.3). For the example embodiment, 1,000 pixels are selected for the upper (2.9) and a similar number for the lower (2.10) region The selection of which pixels to use inside the regions is random in this embodiment but further methods can be used to select the best possible pixels that could reduce the noise on the signal that will be later obtained. The amount of pixels was also chosen for processing efficiency but this can be dynamically defined based on the type of subject, illumination and quality of the images. Once the pixels (3.9) are selected, since they come from a color video image; a gray scale conversion (3.2) is performed so only the luminance component of each pixel will be used for the rest of the process. The method to generate the gray scale conversion (3.2) is taking a percentage of each of the 3 color components to generate a combined signal (for example, 59% of green, 30% red and 11% blue). Using the gray scale is also an alternative that can be changed as the use of only one color component or other combinations of them. The algorithm described in FIG 3, detects the first frame of the image on (3.3) and it is used as a reference frame (3.4).

The reference frame (3.4) is inputted to the pixel tracking algorithm, in our case we used a public domain algorithm called "Lucas-Kanade" (3.6) and in simple terms it compares the position of the pixel (3.9) on reference frame (3.4) with the same pixels of the following frame (4.1) as shown in FIG. 4. Since the subject is moving in X and in Y direction between frame and frame, a group of signals (3.8) that represent the vertical movement (movement of interest in our case) will be outputted by the tracking algorithm (3.6) until the last frame is reached (3.7).

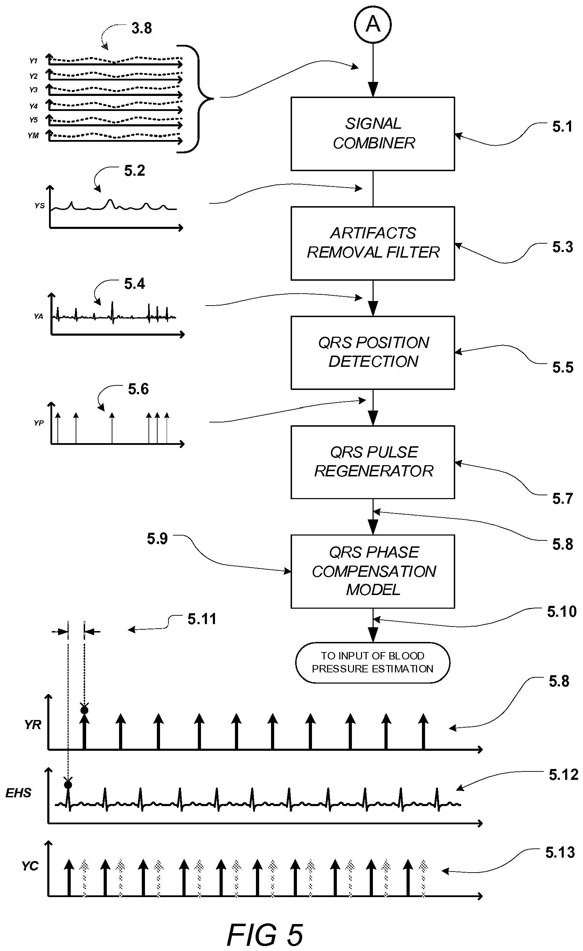

At the end of the process, we will have a plurality of signals (3.8) from Y1 to YM (3.4) as shown in FIG. 4, in our embodiment, the value of M was defined to be 2000. The signals will have the vertical movements (4.0) of the head, (2.11) but since in a normal subject the movement will be very subtle, further processing can be required in order to enhance and eliminate unwanted artifacts (noises) as is described on FIG. 5.

The first step for processing the plurality of signals (3.8) is called the signal combiner (5.1). This functional block obtains a single signal from all the plurality of signals (3.8) related to the vertical head movement. In the example embodiment we used the average of all the signals. That is, we used the vertical position on a particular time, added all the values obtained for that time in all signals and divided the resultant value in the amount of signals (2000 in our embodiment) to obtain a single value for that particular time (5.2). Other methods for combining the signals can be used like auto-correlation or cross-correlation in order to enhance or improve the signal depending on the subject and on the image characteristics.

Even if the subject (1.1) remains physically still, there are other components not related to the heart that will be present on the combined signal (5.2). These components are called artifacts and are caused by breathing, eye blinking, eye movements and involuntary face and neck movements. The later artifacts combine with the signal of interest (5.12) by a process called inter-modulation. The later means that signal (5.2) contains components on other frequencies that distort the signal of interest so the removal of these artifacts is required (5.3).

There are many methods to eliminate unwanted components, in our embodiment, the block (5.3) was implemented using Empirical Mode Decomposition or (EMD). The EMD technique decomposes the signal in the time domain to form a plurality of signals that are orthogonal and non-related, this way we can eliminate the signals that have frequency components not related with the heart electrical signal (2.12). The benefit of a time domain decomposition of the signal is that it does not have a major effect on the phase of the signals as with conventional frequency domain filtering. It is a filter intended for nonlinear and non-stationary signals as in our case. Other techniques already developed like "wavelet filtering" can be used to serve the same purpose of artifact removal (5.3).

At the output of (5.3) we will have a signal (5.4) that resembles the signal of interest (2.12). In the example embodiment, we desire a signal that indicates the position of the QRS (2.13) region in the heart electrical signal (2,12). However, since we can have other artifacts that are random in nature, like a sudden movement of higher intensity, deficiencies on the stability of the light source (1.3) or plain random noise, the signal (5.4) derived at the output of (5.2) can have variations in amplitude and shape that are no longer useful to the (2.4) process. For this point forward, the algorithm is only interested in the position in time of the QRS pulse, and not on the shape and detail of the (5.4) signal.

The next step is called QRS position detection (5.5) and is focused solely on the position in time of the QRS region (2.13). A wavelet based algorithm is used to detect discontinuities on the (5.4) signals and only outputs the time position of these discontinuities as shown in (5.6) where an arrow shows the position in time of the discontinuity that occurred and thus the peak of the QRS signal. It is important to notice that since we are working with signals of very subtle movements, even at this stage we will have spurious and missed pulses from the perspective of the real electrical heart signal that even when it is non-stationary, has a well defined pattern and occurs with regular time base. The block called QRS pulse re-generator performs the analysis of signal (5.6) and based on the frequency and position of the pulses, performs a restoration of missed pulses and also eliminates spurious ones in order to obtain a pulse signal YR (5.8) that includes all (or the majority) of pulses that must be present as in the electrical heart signal (2.12) shown in a larger time frame on (5.12). This QRS pulse re-generator (5.7) can be an optional item if a continuous blood pressure estimation is required.

The circulatory system has an inherent delay between the heart electrical signal (5.12) and the time the head movement (4.0) peaks and valleys occur. The later has an effect of a phase shift (5.11) between the real electrical heart signal (5.2) (as when is obtained with an Electrocardiography equipment or ECG) and the regenerated QRS pulse position signal (5.8). It will be later described the importance to reduce this phase shift (5.11) between the two signals (5.8) and (5.12) to a minimum and this is performed using the phase compensation model (5.9). The processing (5.9) is only required if an ECG with the original time position is needed but does not impact the rest of the process if the step is omitted.

There are several methods for implementing a phase compensation model (5.9). In the example embodiment, we used a relatively simple method that requires having several ECG's from the same subject (1.1) and also several image processing sessions to generate signal (5.8). An average value of phase shift (5.11) is used to generate a constant that when applied to (5.8) compensates the phase shift (5.11) to generate the YC signal (5.13) that follows the heart electrical signal (5.12) with a minimum phase shift deviation (5.11). This method requires a calibration that involves the collection of at least one ECG and that the phase correction factor would only be valid to that particular subject (1.1). The latter will be only required one time and for future blood pressure estimations sessions of the same subject (1.1) the compensating constant will be valid for a certain amount of time until a new recalibration is required. It will also be evident that other types of models that use physical information like height, size, corporal grease density, age, gender, race and skin color etc. can be used to derive a trained model from a statistical population using regression and artificial intelligent techniques, this way, using the information inputted before the analysis of a particular subject (1.1) or could be even totally or partially detected by other image processing algorithms. This input information will suffice to compensate the phase of any new user without previously collecting an ECG.

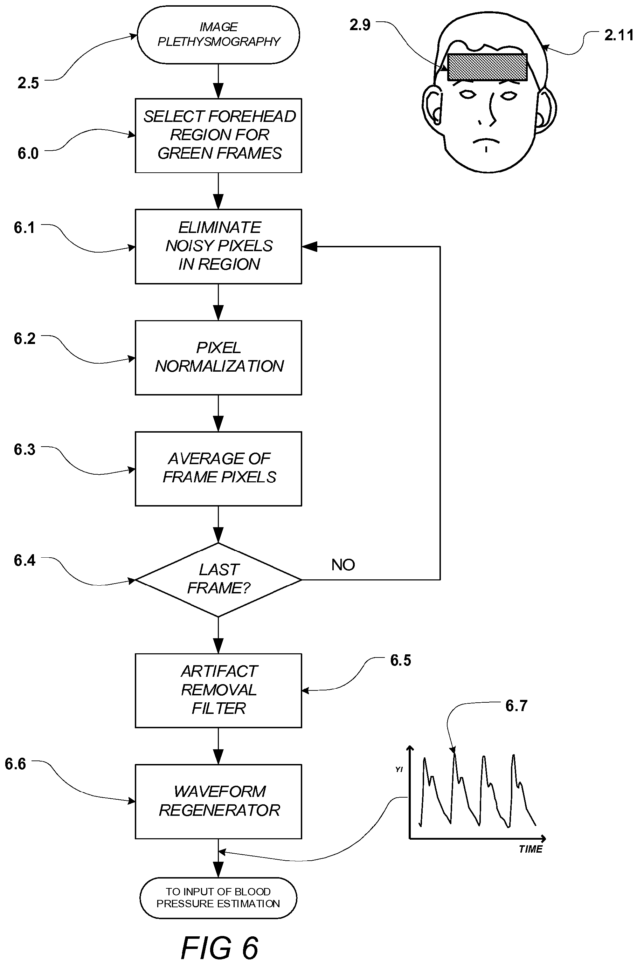

In parallel to the estimation of the QRS pulse position (2.4), the process of image plethysmography (2.5) is executed as shown in FIG. 2. The image plethysmography is described in FIG. 6. This procedure starts with the selection of the region on the upper side of the eyes (2.9) in the subject head (2.1). For this embodiment this particular region is used (6.0) since the pixels contained have less variation between them and this region was already defined by (2.2) described in FIG 2. The latter does not limit to use other regions or even regions of the head (2.1) or even of other parts of the body like an arm, the palm of the hand, a thumb etc. using an additional, or same, video input for this purpose.

Unlike the (2.4) process, instead of generating a gray scale image from the subject (1.1), the image plethysmography process (2.5) uses the green component of the color image since the method that is used relies on the reflection of light and it is the green component that offers the greatest light intensity variations. The latter is because the video cameras usually enhance this particular component to mimic as much as possible the human eye wavelength versus brightness response and this response peaks at the green wavelength region.

The next stage of the process (6.1) consists in the elimination of noisy pixels inside the defined region (2.9). For this, the pixels are compared individually between frame and frame and those that show a high variance in intensity between frames (or groups of them) are discarded, the average percentage of useful pixels finally used can be around the 75% to 80% of the entire region (2.9).

The useful pixels that remain inside the region (2.9) are normalized in (6.2). This process is equivalent to the elimination of the direct current component from the signal and also provides an relative enhancement of the bright intensity of the pixel that would be the equivalent of a gain or amplification The normalized pixel values inside the region for that frame (2.8) are averaged using the arithmetic mean in order to obtain a single value for the (2.9) region that represents the light intensity reflected by the subject (1.1) at that particular time. The arithmetic mean provides also a first stage of filtering since it reduces the amount of noise in the resulting signal, but other mathematical or statistical processing can also be used instead of the average if this produces a best representation of the signal. The processes 6.1 to 6.3 are performed on the frame buffer (2.1) until the last frame is detected by (6.4).

As in the estimation of the QRS position process (2.4) the signal at the output of (6.3) will contain the same artifacts already described. The removal of these unwanted signal elements are carried out by the artifact removal filter (6.5) that for this embodiment also employs the EMD technique to avoid phase alterations but other methods can also be employed.

Given that for this process, the shape of the final signal that will be obtained is of relatively greater importance and considering that this signal will also be subject to missed or spurious cycles, a waveform re-generator (6.6) is also desired. Filters like DF1, DF2, FS2 or "Aya Matsuyama" can be applied to re-generate the signal in order to provide a continuous in time plethysmography signal (6.7) that contains information about the maximum and minimum blood flow versus time on the subject (1.1)

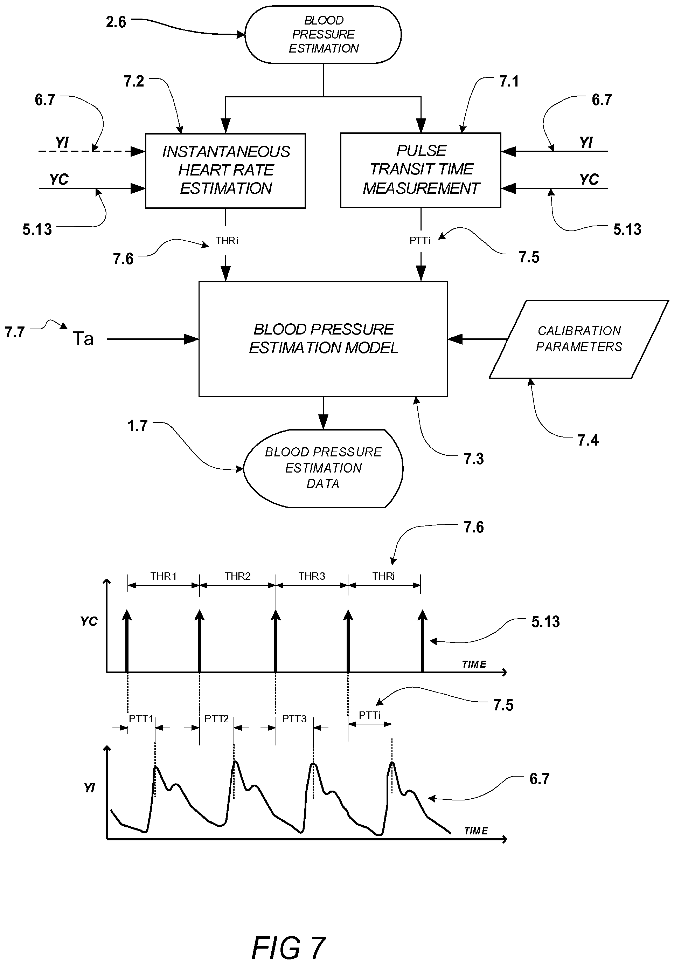

The last stage of the process, used to obtain the blood pressure estimation data (1.7), is described in FIG. 7. The instantaneous heart rate estimation (7.2) takes the phase compensated QRS pulse position signal (5.13) in order to measure the instantaneous heart rate period THRi (7.6). Since the plethysmography signal (6.7) carries the same timing information, signal YI (6.7) can also be used to measure time or to complement the information obtained from YC (5.13).

The pulse transit time measurement (7.1) uses both YI (6.7) and YC (5.13) to measure the time difference between the QRS pulse position and the peak of the plethysmography signal or YI (6.7). The measured time is called pulse transit time PTTi (7.5) and is an important element used to obtain the blood pressure estimation data (1.7).

The instantaneous measurements of time THRi (7.6), the pulse transit time PTTi (7.5), the calibration parameters (7.4) and the ambient temperature (7.7) are fed to the blood pressure estimation model (7.3) that uses this information to derive the blood pressure estimation data (1.7) as it will further detailed.

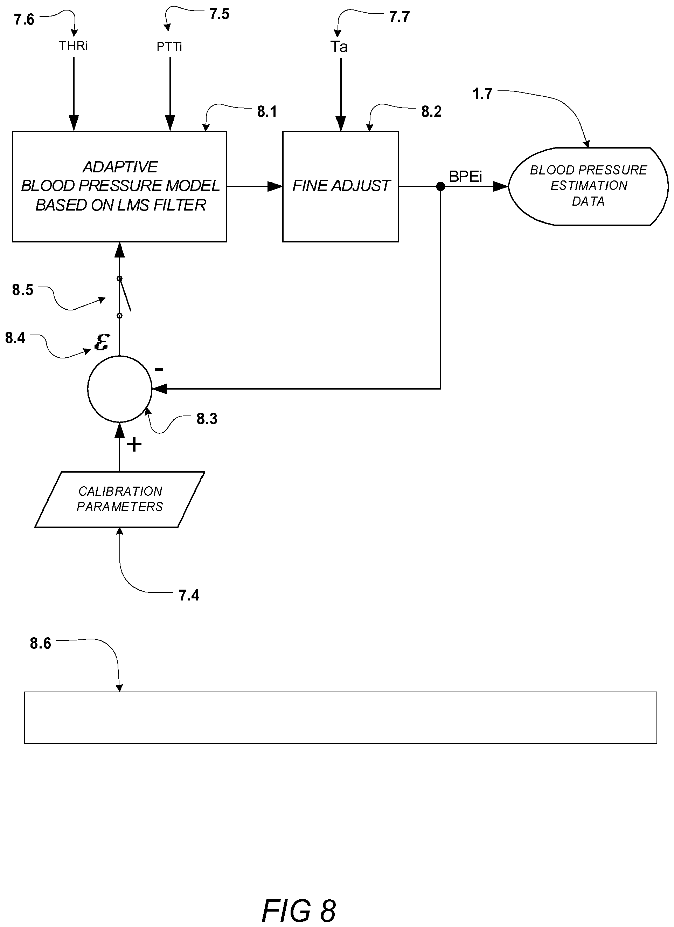

A more detailed description for the blood pressure estimation model is shown in FIG. 8. When a model for this type of application is designed, there are two popular approaches to use: one is called the "maximum likelihood" (MLE), the other is the use of an "adaptive" model. For this example embodiment the adaptive model was employed. The adaptive model can be implemented using several alternatives of algorithms like the Kalman filter, the root mean squared filter (RMS) or least mean squares filter (LMS). For this example embodiment, we will use the LMS approach since it uses only multiplications, subtractions, and additions and this implies an ease of implementation on the image processing systems (1.6) since it requires fewer computational resources and this factor can be important if a real time implementation is required.

One of the advantages of using an adaptive model is that small variations in the two input variables THRi and PTTi are constantly corrected so error in the estimation is reduced. At the output of the adaptive model (8.1) there is another correction element called the fine adjust (8.2). This element takes into account the ambient temperature and compensates in cases when the temperature of the analysis is very different than the temperature when the calibration process was performed since blood pressure tends to rise at lower temperatures and decrease at higher temperatures. Other environmental factors can be taken into account in order to derive a more precise blood pressure estimation data (1.7).

The adaptive model (8.1) for this embodiment can require a calibration process in the same fashion as the phase compensation model (5.9). When the calibration process is performed, the signal switch (8.5) is closed and the model is fed with the calibration parameters (7.4) that are basically the real measured pulse transit time (PTTm), heart rate period (THRm), systolic blood pressure (SBPm) and diastolic blood pressure (DBPm). The parameters are compared with the ones obtained by the image processing so the algorithm in (8.1) is calibrated to minimize the error (8.4). Once the adaptive model (8.1) is calibrated for that subject, the calibration will be valid for this particular subject on subsequent sessions without the need of the real measured data so the switch (8.5) is in the "off"" position during this sessions. In simple terms, the blood estimation model (7.3) is performing a linear approximation as described in (8.6), where the constants C0, C1, C2 are obtained during the calibration process. The temperature compensation KF performed in (8.2) can be obtained also by knowing a set of data from a particular subject or using other types of relations obtained from the general population. As for the phase compensation model (5.9), the data from a plurality of individuals can be utilized and a more complex learning type algorithm can be used in (8.1) in other embodiments so this general model can be applied to any subject without previous calibration that was required for the particular subject in the preferred embodiment.

Example Embodiment. An example embodiment of a method comprises the following steps. Video is captured of the face of a subject, for example 300 frames over 10 seconds, at 1280x720 resolution. Face recognition and tracking software is used to allow retention in each frame of only the portions of the images that pertain to the subject's head, and to remove the eyes from the image. The face regions (above and below the eyes) are identified in the first frame.

The QRS pulse can be estimated from a plurality of frames (e.g., all frames can be used), according to the following. 100 pixels from above the eyes and 1000 pixels from below the eyes can be selected. The color image can be converted to grey scale, e.g., 59% green, 30% red, 11% blue. A first frame can be identified as a reference. A Lucas-Kanade method can be used to track pixels, and vertical movement determined from comparison of other frames to the reference frame. The pixel movement can be combined, e.g., by the sum of the vertical position/2000 pixels. Movement artifacts can be removed, e.g., by empirical mode decomposition such as filtering. The position of the QRS pulse can be determined, e.g., by wavelet decomposition or correlation such as detection of the energy peak. A statistical mode or learning machine can be used to regenerate the QRS pulse. A statistical phase composition model can be used for phase compensation. The QRS timing is then known.

Image plethysmography can be applied to a plurality of frames (e.g., all frames can be used) after face regions have been identified. Forehead pixels can be selected, e.g., by selecting green pixels. Noisy pixels can be eliminated, e.g., by rank order of pixels. Pixels can be normalized, e.g., by removing the average value. Movement artifacts can be removed, e.g., my empirical mode decomposition such as filtering. The waveform can be regenerated, e.g. by a AYA Matsuyama filter.

The blood pressure of the subject can be determined. The heart rate period can be determined from the time difference between the peak of the QRS pulse position signal. The pulse transit time can be determined from the time difference between the QRS pulse position and the peak of the plethysmography signal. The blood pressure can be determined from those, e.g., by an adaptive model such as least mean squares, or a learning machine.

## Claims

1. A method of determining blood pressure of a patient comprising (a) collecting a plurality of images of the head of the patient (1.1) using a camera (1.4), and (b) determining from the plurality of images a measure of the blood pressure of the patient (1.1), without requiring physical contact with the patient;
wherein step (b) comprises: (b1) determining, from the plurality of images, time points corresponding to the QRS cycle of the patient's circulatory system, (b2) determining from the images and the time points a heart rate estimation and a pulse transit time measurement, and (b3) determining the blood pressure from the heart rate estimation and the pulse transit time measurement using a blood pressure estimation model calibrated with previously recorded measured pulse transit time, heart rate period, systolic blood pressure, and diastolic blood pressure.

2. A method as in claim 1, wherein step (b) comprises determining a measure of blood pressure using a model comprising at least one of: a model calibrated statistically through clinical trials or mass measurements, and a model calibrated for each subject using reference instruments.

3. A method as in claim 1, wherein step (b) comprises determining from the plurality of images time points corresponding to the QRS cycle of the patient (1.1).

4. An apparatus for the determination of blood pressure in a patient (1.1), comprising: (a) an image capture system (1.4), configured to capture a plurality of images of the head of the patient; (b) an analysis system (1.6), configured to determine from the plurality of images a measure of the blood pressure of the patient; wherein the analysis system (1.6) is configured to determine, from the plurality of images, time points corresponding to the QRS cycle of the patient's circulatory system, determine from the images and the time points a heart rate estimation and a pulse transit time measurement, and determine the blood pressure from the heart rate estimation and the pulse transit time measurement using a blood pressure estimation model calibrated with previously recorded measured pulse transit time, heart rate period, systolic blood pressure, and diastolic blood pressure.

5. An apparatus as in claim 4, wherein the apparatus is configured to mount in a location where long term monitoring of a patient (1.1) is possible.

6. An apparatus as in claim 5, wherein the apparatus is configured to mount with a car or a television, cell phone, a remote medical monitor station, or a system designed to monitor elderly for aging in place.

7. An apparatus as in claim 4, wherein the apparatus is configured to mount in a location where periodic monitoring is possible (1.1).

8. A method as claimed in claim 1, comprising: (a) capturing video of the subject's head; (b) determining a QRS pulse from the video; (c) determining a plethysmography signal (6.7) from the video; and (d) determining the pulse transit time measurement from the QRS pulse and the plethysmography signal.

9. A method as in claim 8, wherein capturing video comprises collecting a plurality of images of the subject's head at an image capture rate of 300 frames in about 10 seconds.

10. A method as in claim 8, wherein capturing video comprises defining regions of interest in each frame of the video, wherein the regions of interest comprise regions above and below the eyes of the subject.

11. A method as in claim 8, wherein determining a pulse transit time comprises determining a time difference between the position of the QRS pulse and a peak of the plethysmography signal (6.7).

12. A method as in claim 1, further comprising receiving a measure of ambient temperature, and wherein step (b) comprises determining the blood pressure from the measure of ambient temperature, the heart rate estimation and the pulse transit time measurement using a blood pressure estimation model calibrated with previously recorded measured pulse transit time, heart rate period, systolic blood pressure, and diastolic blood pressure.

## Patentansprüche

1. Verfahren zum Bestimmen des Blutdrucks eines Patienten, umfassend (a) Sammeln einer Vielzahl von Bildern des Kopfes des Patienten (1.1) unter Verwendung einer Kamera (1.4) und (b) Bestimmen eines Maßes des Blutdrucks des Patienten (1.1) aus der Vielzahl von Bildern, ohne dass physischer Kontakt mit dem Patienten erforderlich ist;
wobei Schritt (b) umfasst: (b1) Bestimmen von Zeitpunkten, die dem QRS-Zyklus des Kreislaufsystems des Patienten entsprechen, aus der Vielzahl der Bilder, (b2) Bestimmen einer Herzfrequenzschätzung und einer Pulswellenlaufzeitmessung aus den Bildern und den Zeitpunkten, und (b3) Bestimmen des Blutdrucks aus der Herzfrequenzschätzung und der Pulswellenlaufzeitmessung unter Verwendung eines Blutdruckschätzungsmodells, das mit zuvor aufgezeichneter gemessener Pulswellenlaufzeit, Herzfrequenzperiode, zuvor aufgezeichnetem gemessenem systolischem Blutdrucks und diastolischem Blutdruck kalibriert wurde.

2. Verfahren nach Anspruch 1, wobei Schritt (b) Bestimmen eines Maßes des Blutdrucks unter Verwendung eines Modells umfasst, das mindestens eines von einem Modell, das statistisch durch klinische Versuche oder Massenmessungen kalibriert wurde, und einem Modell ist, das für jedes Subjekt unter Verwendung von Referenzelementen kalibriert wurde.

3. Verfahren nach Anspruch 1, wobei Schritt (b) Bestimmen von Zeitpunkten, die dem QRS-Zyklus des Patienten (1.1) entsprechen, aus der Vielzahl der Bilder umfasst.

4. Vorrichtung zur Bestimmung des Blutdrucks in einem Patienten (1.1), umfassend: (a) ein Bilderfassungssystem (1.4), das ausgestaltet ist, um eine Vielzahl von Bildern des Kopfes des Patienten zu erfassen; (b) ein Analysesystem (1.6), das ausgestaltet ist, um aus der Vielzahl von Bildern ein Maß des Blutdrucks des Patienten zu bestimmen; wobei das Analysesystem (1.6) ausgestaltet ist, um aus der Vielzahl von Bildern Zeitpunkte zu bestimmen, die dem QRS-Zyklus des Kreislaufsystems des Patienten entsprechen, aus den Bildern und den Zeitpunkten eine Herzfrequenzschätzung und eine Pulswellenlaufzeitmessung zu bestimmen, und aus der Herzfrequenzschätzung und der Pulswellenlaufzeitmessung unter Verwendung eines Blutdruckschätzungsmodells, das mit zuvor aufgezeichneter gemessener Pulswellenlaufzeit, Herzfrequenzperiode, zuvor aufgezeichnetem gemessenem systolischem Blutdruck und diastolischem Blutdruck kalibriert wurde, den Blutdruck zu bestimmen.

5. Vorrichtung nach Anspruch 4, wobei die Vorrichtung ausgestaltet ist, um an einem Ort montiert zu werden, an dem langfristige Überwachung eines Patienten (1.1) möglich ist.

6. Vorrichtung nach Anspruch 5, wobei die Vorrichtung ausgestaltet ist, um mit einem Wagen oder einem Fernseher, einem Mobiltelefon, einer medizinischen Fernüberwachungsstation oder einem System montiert zu werden, das zur Überwachung von älteren Personen während des Alterungsprozesses vor Ort konzipiert ist.

7. Vorrichtung nach Anspruch 4, wobei die Vorrichtung ausgestaltet ist, um an einem Ort montiert zu werden, an dem periodische Überwachung möglich ist (1.1).

8. Verfahren nach Anspruch 1, umfassend: (a) Erfassen eines Videos von dem Kopf des Subjekts; (b) Bestimmen eines QRS-Pulses aus dem Video; (c) Bestimmen eines Plethysmographiesignals (6.7) aus dem Video; und (d) Bestimmen der Pulswellenlaufzeitmessung aus dem QRS-Puls und dem Plethysmographiesignal.

9. Verfahren nach Anspruch 8, wobei Erfassen des Videos Sammeln einer Vielzahl von Bildern des Kopfes des Subjekts mit einer Bilderfassungsrate von 300 Einzelbildern (Frames) in etwa 10 Sekunden umfasst.

10. Verfahren nach Anspruch 8, wobei das Erfassen des Videos Definieren von interessierenden Regionen in jedem Frame des Videos umfasst, wobei die interessierenden Regionen Regionen oberhalb und unterhalb der Augen des Subjekts umfassen.

11. Verfahren nach Anspruch 8, wobei Bestimmen einer Pulswellenlaufzeit Bestimmen einer Zeitdifferenz zwischen der Position des QRS-Pulses und einem Peak des Plethysmographiesignals (6.7) umfasst.

12. Verfahren nach Anspruch 1, des Weiteren umfassend Empfangen eines Maßes der Umgebungstemperatur, und wobei Schritt (b) Bestimmen des Blutdrucks aus dem Maß der Umgebungstemperatur, der Herzfrequenzschätzung und der Pulswellenlaufzeitmessung unter Verwendung eines Blutdruckschätzungsmodells umfasst, das mit zuvor aufgezeichneter gemessener Pulswellenlaufzeit, Herzfrequenzperiode, zuvor aufgezeichnetem gemessenem systolischem Blutdruck und diastolischem Blutdruck kalibriert wurde.

## Revendications

1. Procédé de détermination de la pression sanguine d'un patient comprenant (a) la collecte d'une pluralité d'images de la tête du patient (1.1) en utilisant une caméra (1.4), et (b) la détermination à partir de la pluralité d'images d'une mesure de la pression sanguine du patient (1.1), sans nécessiter de contact physique avec le patient ;
l'étape (b) comprenant : (b1) la détermination, à partir de la pluralité d'images, de points temporels correspondant au cycle QRS du système circulatoire du patient, (b2) la détermination, à partir des images et des points temporels, d'une estimation de la fréquence cardiaque et d'une mesure du temps de transit du pouls, et (b3) la détermination de la pression sanguine à partir de l'estimation de la fréquence cardiaque et de la mesure du temps de transit du pouls en utilisant un modèle d'estimation de la pression sanguine étalonné avec le temps de transit du pouls, la période de la fréquence cardiaque, la pression sanguine systolique et la pression sanguine diastolique mesurés précédemment.

2. Procédé selon la revendication 1, l'étape (b) comprenant la détermination d'une mesure de la pression sanguine en utilisant un modèle comprenant au moins l'un des éléments suivants : un modèle étalonné statistiquement par des essais cliniques ou des mesures de masse, et un modèle étalonné pour chaque sujet en utilisant des instruments de référence.

3. Procédé selon la revendication 1, l'étape (b) comprenant la détermination à partir de la pluralité d'images de points temporels correspondant au cycle QRS du patient (1.1).

4. Appareil pour la détermination de la pression sanguine chez un patient (1.1), comprenant :
(a) un système de capture d'images (1.4), configuré pour capturer une pluralité d'images de la tête du patient ;
(b) un système d'analyse (1.6), configuré pour déterminer à partir de la pluralité d'images une mesure de la pression sanguine du patient ; le système d'analyse (1.6) étant configuré pour déterminer, à partir de la pluralité d'images, des points temporels correspondant au cycle QRS du système circulatoire du patient, déterminer à partir des images et des points temporels une estimation de la fréquence cardiaque et une mesure du temps de transit du pouls, et déterminer la pression sanguine à partir de l'estimation de la fréquence cardiaque et de la mesure du temps de transit du pouls en utilisant un modèle d'estimation de la pression sanguine étalonné avec le temps de transit du pouls, la période de la fréquence cardiaque, la pression sanguine systolique et la pression sanguine diastolique mesurés précédemment.

5. Appareil selon la revendication 4, l'appareil étant configuré pour être monté dans un endroit où une surveillance à long terme d'un patient (1.1) est possible.

6. Appareil selon la revendication 5, l'appareil étant configuré pour être monté avec une voiture ou un téléviseur, un téléphone portable, une station de surveillance médicale à distance, ou un système conçu pour surveiller les personnes âgées pour le vieillissement chez soi.

7. Appareil selon la revendication 4, l'appareil étant configuré pour être monté dans un endroit où une surveillance périodique est possible (1.1).

8. Procédé selon la revendication 1, comprenant : (a) la capture d'une vidéo de la tête du sujet ; (b) la détermination d'une impulsion QRS à partir de la vidéo ; (c) la détermination d'un signal pléthysmographique (6.7) à partir de la vidéo ; et (d) la détermination de la mesure du temps de transit du pouls à partir de l'impulsion QRS et du signal pléthysmographique.

9. Procédé selon la revendication 8, la capture vidéo comprenant la collecte d'une pluralité d'images de la tête du sujet à une fréquence de capture d'images de 300 images en environ 10 secondes.

10. Procédé selon la revendication 8, la capture vidéo comprenant la définition de régions d'intérêt dans chaque image de la vidéo, les régions d'intérêt comprenant des régions au-dessus et au-dessous des yeux du sujet.

11. Procédé selon la revendication 8, la détermination d'un temps de transit du pouls comprenant la détermination d'une différence de temps entre la position de l'impulsion QRS et un pic du signal pléthysmographique (6.7).

12. Procédé selon la revendication 1, comprenant en outre la réception d'une mesure de la température ambiante, et l'étape (b) comprenant la détermination de la pression sanguine à partir de la mesure de la température ambiante, de l'estimation de la fréquence cardiaque et de la mesure du temps de transit du pouls en utilisant un modèle d'estimation de la pression sanguine étalonné avec le temps de transit du pouls, la période de la fréquence cardiaque, la pression sanguine systolique et la pression sanguine diastolique mesurés précédemment.
